# EUROPEAN PATENT APPLICATION

(11) **EP 1 352 616 A1**
(43) Date of publication of application: **15.10.2003**
(21) Application number: 02025913.1
(22) Date of filing: 20.11.2002
(51) Int. Cl.: A61B 19/04, C08K 3/06, C08K 3/22

(54) **Soft nitrile medical gloves having improved glove relaxation properties**

(30) Priority: 12.04.2002 US 120796
(71) Applicant: Microflex Corporation, Reno, Nevada 89523 (US)
(72) Inventor: Tao, Jian, Reno, Nevada 89523 (US)
(74) Representative: Fritz, Edmund Lothar, Dipl.-Chem.

(57) **Abstract**

The invention is the manipulation of the zinc oxide content and sulfur content of nitrile butadiene rubbers and selected vulcanization conditions that can be achieved economically with common production facilities. The manipulation of these components affect the relaxation property of gloves formed by this material. Produced are gloves that have a relaxation property, higher than 50%, and a low modulus (approximately 3 Mpa). The glove maintains decent ultimate tensile strength (> 20 Mpa) and elongation (> 500%). The glove is produced by a vulcanization process which lasts from 5 to 60 minutes at temperatures ranging from 300°F to 400°F. The tensile strength and elongation are well above the ASTM requirements for medical gloves. The current ASTM requirements are ASTM D412-92. Thanks to sufficient vulcanization, the films produced provide satisfactory protection from viral penetration. The tearing strength is also better because of the lower modulus.

## Description

### BACKGROUND OF THE INVENTION

Natural rubber latex gloves provide excellent protection from numerous dangerous pathogens as well as many harsh chemicals. The natural rubber latex glove manufacturing industry mushroomed in early 1980s, especially in the Far East. However, soon after that, it was recognized that the inherent proteins of natural rubber latex would cause allergic reactions (Type I) to occur in certain people. In rare cases, the allergic reaction could be fatal. Therefore, for those people, alternatives, to natural rubber latex gloves, must be provided.

Although a series of synthetic materials including nitrile butadiene rubber (NBR), polychloroprene (CR), polyurethane (PU), polyisoprene (IR), polyvinyl chloride (vinyl, PVC), polyethylene (PE), etc. as well as many of their blends and copolymers have been used as alternatives to natural rubber latex, the overall performance and the cost of the alternatives are not quite satisfactory. Among the alternatives, nitrile butadiene rubber is the most popular one, an elastic glove at a reasonable cost.

U.S. Patent 5,014,312, and Reissue Patent RE 35,616, both issued to Tillotson et al, cover nitrile butadiene rubber gloves. The patents address relaxation properties. The stress (or modulus) of the material under constant strain at six minutes should be less than 50% of its initial value. Most of the nitrile gloves currently commercially available have their relaxation property clustered about 40%, although that could be varying from 30 ∼ 45%. Other gloves might have improved tensile strength, or elongation, or fewer additives that could cause Type IV allergic reactions (ZnO, etc.). None of them have displayed relaxations at six minutes that could exceed 50%.

Relaxation property is not an ASTM required quality control parameter for gloves. But together with modulus, another non-ASTM required quality control parameter for gloves; they can characterize the performance and the tactile sensation of a glove. The higher the relaxation property, the better the glove will fit a hand's shape. Otherwise, the glove becomes loose after a while. But if high relaxation is combined with high modulus, the glove would quickly cause finger fatigue. Natural rubber latex gloves has a (relaxation > 80%, and a 300% modulus (< 2 MPa), while nitrile butadiene rubber gloves show lower relaxation (typical 40%) and a much higher modulus at 300% (>7 Mpa).

Relaxation property is an intrinsic characteristic of material nature. Most nitrile butadiene latexes manufactured via emulsion polymerization would yield a relaxation of about 40%, as evidenced by the nitrile gloves currently available. This inherent property is predominantly caused by polymer chain structure, which would be determined by the polymerization mechanism. Different nitrile butadiene rubber vendors might have different controlling parameters and procedures, but their products have very little differentiation due to the fact that they all use emulsion polymerization for economic reasons.

Nitrile butadiene latexes, produced via polymerization mechanisms other than emulsification, namely for dipping applications could have quite different structure, and thus different relaxation profiles, but there are no such products that are commercially available right now because of cost. Once the polymer chain structure has been predefined in the polymerization, there is little one can do to manipulate it. It is an objective of the invention to tune this parameter (relaxation) to above 50%. Meanwhile, the other mechanical properties must meet ASTM requirements.

### SUMMARY OF THE INVENTION

The invention is the manipulation of the zinc oxide content and sulfur content of nitrile butadiene rubbers and selected vulcanization conditions that can be achieved economically with common production facilities. The manipulation of these components affect the relaxation property of gloves formed by this material. Produced are gloves that have a relaxation property, higher than 50%, and a low modulus (approximately 3 Mpa). The glove maintains decent ultimate tensile strength (> 20 Mpa) and elongation (> 500%). The glove is produced by a vulcanization process which lasts from 5 to 60 minutes at temperatures ranging from 300°F to 400°F. The tensile strength and elongation are well above the ASTM requirements for medical gloves. The current ASTM requirements are ASTM D412-92. Thanks to sufficient vulcanization, the films produced provide satisfactory protection from viral penetration. The tearing strength is also better because of the lower modulus.

### DETAILED DESCRIPTION OF THE INVENTION

### Compounding:

Carboxylated nitrile butadiene rubber undergoes two kinds of crosslinking in normal formulation and vulcanization. The first one is where carboxylated acid groups are linked to each other via a reaction with zinc oxide at room temperature. The second kind of crosslinking is where unsaturated butadiene blocks are crosslinked via a conventional sulfur system at elevated temperatures.

When zinc oxide is used, the relaxation property is not very sensitive to variation of zinc oxide content, although other mechanical properties (modulus, ultimate elongation and tensile strength) are favorable. One of our preferred formulations, produce films whose properties are comparable to those of natural rubber latex (300% modulus < 2 Mpa, tensile strength > 20 Mpa, and simultaneously elongation > 500%). But, the relaxation at six minutes can not be higher than 45%. Therefore, zinc oxide was eliminated to prevent a performance reduction. The resulting formulation is tabulated as following:

| **INGREDIENTS** | **PHR** |
|---|---|
| Nitrile Butadiene Rubber | 100 |
| 2,2'-methylene-bis-(4-methyl-6-butylphenol) | 0.5 |
| zinc 2-mercaptobenzothiazole | 1.0 |
| zinc dibutyldithiocarbamate (BZ) | 1.0 |
| sulfur | 3.0 |
| potassium hydroxide | 1.0 |
| titanium dioxide | 0.5 |

### Vulcanization conditions:

Zinc oxide has been used as a primary activator for conventional sulfur vulcanization systems. By eliminating zinc oxide, higher temperatures were used during vulcanization. The films after leaching were dried at 260°F and then vulcanized at 300-400°F for 5-60 minutes. Conventionally, curing temperatures range between 240°F and 285°F.

### Mechanical properties:

A couple of films obtained from the above formulation and vulcanization conditions show the following characteristics:

| | Modulus at 300% (Mpa) | Ultimate Elongation (%) | Tensile Strength (Mpa) | % Initial Press at 6 min |
|---|---|---|---|---|
| Film 1 | 3.11 | 578 | 23.93 | 53.9 |
| Film 2 | 3.20 | 555 | 21.03 | 56.4 |
| Film 3 | 3.83 | 506 | 21.36 | 58.8 |
| Film 4 | 3.77 | 500 | 18.99 | 62.7 |

As a result, we found zinc oxide free formulations and vulcanization conditions that yield higher relaxation properties and an improved soft glove. This combination shows a more balanced performance.

The zinc oxide free compound exhibited relaxation in the neighborhood of 55%. Powdering and/or leaching had no significant impact on glove properties. The formulations produce improved compounds and optimized vulcanization conditions (temperature and duration). The new formulations and procedures are easily realized economically under common nitrile-glove production lines. Depending on the desired applications, both powdered and powder free gloves could be produced in the same formula.

In a generic formula with both zinc oxide and sulfur, two kinds of crosslinking mechanisms govern the carboxylated NBR vulcanization. At room temperature, zinc oxide reacts with carboxylated groups to form ionic crosslinking, resulting in low relaxation. On the other hand, sulfur crosslinking, especially multi-sulfur crosslinking between double bounds of polybutadiene chains results in high relaxation. Varying the ratio of these two components, one can tune the relaxation to a certain level. The previously disclosed zinc oxide free embodiment achieved at relaxation as high as 66%, compared to 40% with normal 1 zinc oxide and 1 sulfur formula. With limited zinc oxide content of 0.5 parts zinc oxide per 100 parts NBR and abnormal 5 parts of sulfur per 100 parts NBR, relaxation of 50% can still be achieved. The affect of the varied ratio of the zinc oxide and sulfur is summarized on the following table:

| **Zinc Oxide Parts Per 100 parts NBR** | **Sulfur Parts Per 100 parts NBR** | **Relaxation (%)** |
|---|---|---|
| 1 | 1 | 40 |
| 0 | 3 | 66.2 |
| 0.5 | 3 | 50.0 |
| 0.3 | 3 | 54.1 |
| 0.2 | 3 | 54.5 |
| 0.1 | 3 | 56.9 |
| | | |
| 0 | 3 | 66.2 |
| 0.5 | 0.3 | 34.5 |
| 0.5 | 0.5 | 39.1 |
| 0.5 | 3 | 50.0 |
| 0.5 | 5 | 60.2 |
| 0 | 5 | 64.4 |

The first row is a normal formulation whereas the second row is the preferred embodiment of the invention. Rows 3-7 illustrate the effect of varying the zinc oxide as each of these rows has a constant sulfur content. As can be seen, lowering the amount of zinc oxide increases the relaxation property. Rows 8-11 show the effect of varying the sulfur as the amount of zinc oxide is held constant and the amount of sulfur is varied. As can be seen here, the increase in sulfur causes an increase in the relaxation property. These elastomers were made under the same vulcanization process disclosed above.

As mentioned previously, relaxation is an intrinsic property of the polymer structure and in carboxylated NBR, the most predominant factor is the chain structure of polybutadiene. In commercially available carboxylated NBR synthesized via emulsion polymerization, the polybutadiene block consists of three types of double bonds: 1,2; cis 1,4; and trans 1,4. More cis 1,4 structure yields high relaxation. It is possible to synthesize polybutadiene with cis 1,4 dominant structures in an organic solvent. In this way, it is possible to achieve higher relaxation, > than 80%, with the normal formula. For an NBR, which is not carboxylated, there is no reaction between the zinc oxide and carboxylated groups and therefore, the content of zinc oxide may not matter. Natural rubber may have a relaxation property > than 80% with one part per 100 of zinc oxide.

While the invention has been described with respect to a preferred embodiment, variations, modifications would be apparent to one of ordinary skill in the art without departing from the spirit of the invention.

## Claims

1. An elastomer formulation for a glove comprising:
a nitrile butadiene rubber latex;
sulfur;
zinc oxide, said zinc oxide, present in amounts up to 0.5 parts per hundred parts nitrile butadiene rubber.

2. The elastomer formulation for a glove as claimed in claim 1, wherein said sulfur is present in amounts of up to five parts per hundred parts nitrile butadiene rubber.

3. The elastomer formulation for a glove as claimed in claim 2, wherein said sulfur is present in three parts per hundred parts nitrile butadiene rubber.

4. The elastomer formulation for a glove as claimed in claim 1, wherein said zinc oxide is present in 0.5 parts per hundred parts nitrile butadiene rubber.

5. The elastomer formulation for a glove as claimed in claim 1, wherein said elastomer has a relaxation property of over 50%.

6. The elastomer formulation for a glove as claimed in claim 1, wherein said elastomer is vulcanized at 300-400 degrees Fahrenheit for 5-60 minutes.

7. An elastomer formulation for a glove comprising:
a nitrile butadiene rubber latex;
sulfur, said sulfur is present in three parts per hundred parts nitrile butadiene rubber;
zinc oxide, said zinc oxide, present in 0.5 parts per hundred parts nitrile butadiene rubber, wherein said elastomer has a relaxation property of over 50% and is vulcanized at 300-400 degrees Fahrenheit for 5-60 minutes.qa.

## Amended claims

### Amended claims in accordance with Rule 86(2) EPC.

**1.** An elastomer formulation for a glove comprising:
a nitrile butadiene rubber latex;
sulfur;
zinc oxide, said zinc oxide, present in amounts up to 0.5 parts per hundred parts nitrile butadiene rubber, wherein said elastomer is vulcanized at 300-400 degrees Fahrenheit for 5-60 minutes.

**2.** The elastomer formulation for a glove as claimed in claim 1, wherein said sulfur is present in amounts of up to five parts per hundred parts nitrile butadiene rubber.

**3.** The elastomer formulation for a glove as claimed in claim 2, wherein said sulfur is present in three parts per hundred parts nitrile butadiene rubber.

**4.** The elastomer formulation for a glove as claimed in claim 1, wherein said zinc oxide is present in 0.5 parts per hundred parts nitrile butadiene rubber.

**5.** The elastomer formulation for a glove as claimed in claim 1, wherein said elastomer has a relaxation property of over 50%.

**6.** An elastomer formulation for a glove comprising:
a nitrile butadiene rubber latex;
sulfur, said sulfur is present in three parts per hundred parts nitrile butadiene rubber;
zinc oxide, said zinc oxide, present in 0.5 parts per hundred parts nitrile butadiene rubber, wherein said elastomer has a relaxation property of over 50% and is vulcanized at 300-400 degrees Fahrenheit for 5-60 minutes.
